# EUROPÄISCHE PATENTSCHRIFT

(11) **EP 3 200 568 B1**
(45) Veröffentlichungstag und Bekanntmachung des Hinweises auf die Patenterteilung: **06.01.2021**
(21) Anmeldenummer: 17151706.3
(22) Anmeldetag: 17.01.2017
(51) Int. Cl.: H05K 1/02, H01R 4/02, H05K 3/32, H01R 43/02, B23K 26/32, B23K 26/28, B23K 26/22, H05K 3/22, H05K 3/40, B23K 101/36, B23K 101/38

(54) **BATTERIEBRÜCKE UND VERFAHREN ZUM AKTIVIEREN EINER ELEKTRONISCHEN VORRICHTUNG**
BATTERY BRIDGE AND METHOD FOR ACTIVATING AN ELECTRONIC DEVICE
ÉLÉMENT DE LIAISON POUR BATTERIE ET PROCÉDÉ D'ACTIVATION D'UN DISPOSITIF ÉLECTRONIQUE

(30) Priorität: 29.01.2016 DE 102016101620
(43) Veröffentlichungstag der Anmeldung: 02.08.2017
(73) Patentinhaber: BIOTRONIK SE & Co. KG, 12359 Berlin (DE)
(72) Erfinder: Henschel, Martin, 13125 Berlin (DE)
(74) Vertreter: Galander, Marcus

(56) Entgegenhaltungen:
- DE-A1-102007 008 549
- DE-A1-102013 108 563
- US-A1- 2013 048 363
- US-B1- 6 740 821

## Beschreibung

Die Erfindung betrifft eine Batteriebrücke für eine elektronische Vorrichtung, vorzugsweise für ein elektronisches Implantat, sowie ein Verfahren zum Aktivieren einer derartigen elektronischen Vorrichtung mit einem elektrischen Schaltkreis.

Als Batteriebrücke wird der bis zuletzt offen bleibende Kontakt eines elektrischen Stromkreises einer elektronischen Vorrichtung, beispielsweise eines elektronischen Implantats, bezeichnet. Wird der offene Kontakt der Batteriebrücke nach erfolgtem Zusammenbau und Test der elektronischen Vorrichtung geschlossen, so wird die Vorrichtung aktiviert, d.h. der der Vorrichtung zugrunde liegende elektrische Schaltkreis permanent geschlossen.

In Bezug auf ein elektronisches Implantat ist eine ordnungsgemäße Aktivierung naturgemäß besonders wichtig, da eine dauerhaft fehlerfreie Funktion des Implantats gewährleistet sein muss. Als elektronische Implantate sind derzeit beispielsweise Herzschrittmacher, Kardioverter-Defibrillatoren, elektronische Medikamentenabgabevorrichtungen oder implantierbare Sensoren (Biomonitore) im Einsatz.

Aufgrund der Anforderung, dass ein elektrischer Schaltkreis für derartige elektronische Vorrichtungen möglichst wenig Raum einnehmen soll, wird dieser derzeit häufig mittels SMT (Surface Mounting Technology, Oberflächenmontage) hergestellt. Bei der Oberflächenmontage werden Bauelemente mittels lötfähiger Anschlussflächen direkt auf eine Leiterplatte gelötet.

Zur Aktivierung einer elektronischen Vorrichtung wurde bisher beispielsweise ein Lötverfahren verwendet, bei dem Zinn mittels eines Lötkolbens aufgebracht wird und den Stromkreis zwischen zwei benachbarten Lötflächen auf der Leiterplatte (Platine) schließt. Der Nachteil dieser herkömmlichen Lösung besteht darin, dass sich Lötverfahren heutzutage nicht prozesssicher im Hinblick auf die kleinen Strukturen, wie sie auf hochkompakten Schaltkreisen realisiert werden, automatisieren lassen, denn der Lötprozess wird durch die Lotmenge, die Löt-Temperatur, das Flussmittel, die Reinheit der Oberflächen und dergleichen beeinflusst. US 2013/048363 A1 offenbart ein derartiges Verfahren mit dazugehöriger Vorrichtung. Es offenbart damit den Oberbegriff des Anspruchs 1.

Ebenso ist bekannt, einen Kontakt des Batteriebandes mittels eines temporären Isolators bis zur Aktivierung isoliert zu halten. Nach Fertigstellung der elektronischen Vorrichtung, bei der die Kontakte geschweißt werden, wird die Vorrichtung über Testpunkte hochgefahren. Nach erfolgreichem Power-Up wird der temporäre Isolator manuell entfernt; das noch nicht angeschweißte Batterieband manuell niedergehalten und mit ein bis zwei Schweißpunkten manuell angeschweißt. Dieses Verfahren hat den Nachteil, dass der Stromkreis durch eine aufwendige Hochfahrvorrichtung aktiviert werden muss, bei der immer ein Anschlussfähnchen der Batterie über einen temporären Isolator isoliert wird. Hierdurch soll das unbeabsichtigte Kontaktieren des Bandes (Prellen des Kontaktes) und somit eine fehlerhafte Initialisierung vermieden werden. Es ist ebenfalls von Nachteil, dass bei diesem Verfahren nach erfolgreicher Initialisierung des Schaltkreises der temporäre Isolator vom Batterieanschlussbändchen manuell entfernt, das Bändchen mit einer Pinzette auf dem Schaltkreis platziert und anschließend auf dem Schweißpad angeschweißt werden muss. Dieser komplexe manuelle Arbeitsschritt ist zeitaufwendig und feinmotorisch anspruchsvoll. Er lässt sich unter wirtschaftlichen Aspekten nicht automatisieren. Zudem muss zusätzliches Material für den temporären Isolator bereitgestellt, zugeführt und positioniert werden. Gleiches gilt für das Anschlussbändchen.

Eine alternative Lösung bestünde darin, den Stromkreis mittels eines Bondverfahrens zu schließen. Hierfür muss Material (z.B. Aluminium-Band/Draht) zugeführt und die Bandenden mittels Reibschweißen oder Laserschweißen angeschweißt werden. Das Bond-Verfahren setzt aufgrund der hohen auftretenden Kräfte beim Niederhalten und Anschweißen des Bandes (Widerstandsschweißen) einen bereits fixierten Innenaufbau des elektrischen Schaltkreises voraus, z.B. durch Einkleben. Dies ist jedoch für elektronische Implantate problematisch, da keine Klebstoffe verwendet werden sollen, um einerseits einen hohen Zeitaufwand für das Aushärten eines Klebers und andererseits Klebstoffausdünstungen zu vermeiden, die sich auf das elektronische Verhalten der Vorrichtung negativ auswirken können.

Eine weitere alternative Realisierungsmöglichkeit für eine Batteriebrücke könnte darin bestehen, den Stromkreis mittels eines Steckkontakts zu schließen. Für elektronische Implantate besteht jedoch die Anforderung, dass der elektronische Schaltkreis über die gesamte Laufzeit des Implantats hinweg, d.h. bis zu 10 Jahre, zuverlässig arbeiten muss. Die Materialermüdung der Federn oder sich über die Zeit verändernde Übergangswiderstände stellen jedoch ein Risiko hinsichtlich der Produktzuverlässigkeit dar. Daher ist die Verwendung von Steckkontakten/Federkontakten für elektronische Vorrichtungen wie elektronische Implantate nicht angezeigt.

Die Aufgabe der vorliegenden Erfindung besteht somit darin, eine Batteriebrücke zu schaffen, mit der ein elektrischer Stromkreis auf einfache, automatisierbare Weise sicher aktiviert werden kann. Hinsichtlich des Verfahrens besteht die Aufgabe darin, ein Verfahren anzugeben, das auf einfache und kostengünstige Weise eine Aktivierung einer elektronischen Vorrichtung ermöglicht.

Die obige Aufgabe wird durch eine Batteriebrücke gelöst, welche ein elektrisch leitendes erstes Kontaktelement, ein elektrisch leitendes zweites Kontaktelement und einen Isolator aufweist, wobei das erste Kontaktelement und das zweite Kontaktelement ein schweißbares Material aufweisen, wobei in einem ersten ("offenen") Zustand der Batteriebrücke das erste Kontaktelement über einen vorgegebenen Luftspalt beabstandet von dem zweiten Kontaktelement angeordnet ist und das erste Kontaktelement durch den Luftspalt und den Isolator elektrisch von dem zweiten Kontaktelement isoliert ist, wobei die Batteriebrücke derart ausgebildet ist, dass sie mittels Verschweißen des ersten Kontaktelements und des zweiten Kontaktelements miteinander in einen zweiten ("geschlossenen") Zustand überführbar ist, bei dem der Luftspalt zwischen dem ersten Kontaktelement und dem zweiten Kontaktelement zumindest abschnittsweise dauerhaft elektrisch leitend geschlossen ist.

Mittels der erfindungsgemäßen Batteriebrücke wird im zweiten (geschlossenen) Zustand ein Schließen eines elektrischen Stromkreises und damit eine Aktivierung der den Stromkreis aufweisenden Vorrichtung bewirkt. Hierfür werden mittels Schweißen das Material des ersten Kontaktelements und das Material des zweiten Kontaktelements im Bereich des Luftspalts miteinander verschmolzen, so dass einerseits an dieser Stelle der Schweißnaht oder des Schweißpunkts der Luftspalt geschlossen ist und andererseits eine elektrisch leitende Verbindung zwischen dem ersten Kontaktelement und dem zweiten Kontaktelement hergestellt ist.

Die erfindungsgemäße Batteriebrücke muss zumindest in dem Bereich der Kontaktelemente, in dem das Verschweißen erfolgt, aus schweißbarem Material bestehen. Beispielsweise kann das erste Kontaktelement und das zweite Kontaktelement zumindest teilweise aus mindestens einem Material der Gruppe enthaltend Nickel, Kupfer, Kupfer-Nickel-Legierungen, Kupfer, Niob, Tantal, Molybdän, Edelstahl, Platin-Legierungen, PalladiumLegierungen und Titan gefertigt sein.

Der Luftspalt weist vorzugsweise eine Breite im Bereich von 10 µm bis 100 µm, besonders bevorzugt im Bereich von 30 µm bis 80 µm, auf. Ein derartiger Luftspalt besitzt einerseits die notwendige isolierende Eigenschaft im ersten (offenen) Zustand und lässt sich andererseits zur Herstellung des zweiten (geschlossenen) Zustands beispielsweise mittels Laserschweißen sicher elektrisch leitend verschließen. Im ersten (offenen) Zustand ist die elektronische Vorrichtung nicht aktiviert.

Der Isolator isoliert das erste Kontaktelement und das zweiten Kontaktelement elektrisch voneinander. Der Isolator dient aber auch zur mechanischen Befestigung des ersten Kontaktelements und des zweiten Kontaktelements. Dafür ist der Isolator vorzugsweise entweder zwischen dem ersten Kontaktelement oder dem zweiten Kontaktelement angeordnet und mit diesen verbunden oder das erste Kontaktelement und das zweite Kontaktelement sind auf dem Isolator angeordnet und an diesem befestigt.

Die erfindungsgemäße Batteriebrücke zeichnet sich dadurch aus, dass sie einerseits kostengünstig herstellbar ist und andererseits mittels des Schweißprozesses eine automatisierte Aktivierung der elektronischen Vorrichtung ermöglicht. Bei Verwendung einer erfindungsgemäßen Batteriebrücke kann daher in ein schlanker, automatisierter Fertigungsablauf hinsichtlich der Aktivierung/Initialisierung realisiert werden.

Es ist weiter von Vorteil, wenn der Isolator im Wesentlichen ringförmig, das erste Kontaktelement im Querschnitt im Wesentlichen U-förmig oder hohlzylinderförmig und das zweite Kontaktelement im Wesentlichen zylinderförmig ausgebildet ist, wobei der ringförmige Isolator zwischen dem ersten Kontaktelement und dem zweiten Kontaktelement angeordnet ist. Das U-förmige oder hohlzylinderförmige erste Kontaktelement wird auch als Kappe und das zweite zylinderförmige Kontaktelement als Stempel bezeichnet. Ein derartiges rotationssymmetrisches Design der Komponenten führt dazu, dass sich diese selbsttätig zentrieren und so der geringe Isolationsabstand entlang der gesamten Schweißkante sicher realisiert werden kann. Der Isolator hält die elektrisch leitenden, vorzugsweise metallischen Kontaktelemente der Batteriebrücke in Position und gewährleistet den vorgegebenen Luftspalt, der die Isolation der Kontaktelemente voneinander bewirkt. Somit kann ein versehentlicher Kontakt zwischen dem ersten Kontaktelement und dem zweiten Kontaktelement vermieden werden. Eine hinsichtlich des Platzbedarfs besonders vorteilhafte Anordnung der Komponenten ergibt sich dadurch, dass das zweite Kontaktelement innerhalb einer innenliegenden Öffnung des ersten Kontaktelements, welche vorzugsweise durchgehend ist, angeordnet ist, besonders bevorzugt in einem Presssitz.

Es ist insbesondere hinsichtlich des oben erläuterten rotationssymmetrischen Ausführungsbeispiels weiter von Vorteil, wenn das erste Kontaktelement und/oder das zweite Kontaktelement aus einem metallischen Material mittels eines Kaltverformungs- oder Stanzprozesses hergestellt sind. Der Isolator kann beispielsweise als Kunststoffspritzgussteil ausgeführt sein, beispielsweise aufweisend ein Material der Gruppe enthaltend POM (Polyoxymethylen), PEEK (Polyetheretherketon), LCP (Flüssigkristallpolymer) und PBT (Polybutylenterephthalat), oder als Folie aufweisend ein Material der Gruppe enthaltend PEEK, Polyimid, LCP und Polyarylat.

In einem weiteren bevorzugten Ausführungsbeispiel weist das zweite Kontaktelement, vorzugsweise auch das erste Kontaktelement, einen abgeschrägten Abschnitt auf, welcher ein zum Verschweißen verwendetes Laserlicht von einem unterhalb der Batteriebrücke angeordneten elektrischen Schaltkreis, beispielsweise einer Leiterplatte, abschirmt. Der abgeschrägte Abschnitt zeichnet sich dadurch aus, dass in diesen Abschnitt insbesondere bei einer rotationssymmetrischen Ausbildung des ersten Kontaktelements oder des zweiten Kontaktelements eine Änderung des Durchmessers des jeweiligen Kontaktelements von einem kleineren Durchmesser zu einem größeren Durchmesser erfolgt, so dass das Kontaktelement eine schräge Kante ausbildet. Durch diesen abgeschrägten Abschnitt wird das Laserlicht abgelenkt und in eine Richtung weg von einem darunter liegenden elektrischen Schaltkreis reflektiert, so dass eine Beschädigung des Schaltkreises durch das Verschweißen des ersten Kontaktelements mit dem zweiten Kontaktelement vermieden wird.

In einer weiteren bevorzugten Ausführungsform weist das erste Kontaktelement und/oder das zweite Kontaktelement in einem zur Verbindung mit dem elektrischen Schaltkreis dienenden Abschnitt eine die Lötbarkeit verbessernde Beschichtung auf, welche beispielsweise Gold (ENIG) und/oder Palladium enthält. Hierdurch wird die Lötverbindung der Batteriebrücke mit entsprechenden Kontaktflächen von Leiterbahnen einer Leiterplatte, beispielsweise während einer Oberflächenmontage, verbessert.

Es ist weiter von Vorteil, wenn das zweite Kontaktelement im Bereich einer oberen Stirnfläche des ersten Kontaktelements, auf der das Verschweißen erfolgt, über diese obere Stirnfläche hinausragt. Durch diese Gestaltung der erfindungsgemäßen Batteriebrücke wird ein besseres Verschweißen zum Verschließen des Luftspalts zwischen dem ersten Kontaktelement und dem zweiten Kontaktelement, beispielsweise mittels Kehlnahtschweißen, erreicht. Die erzeugte Schweißnaht kann einfacher in Bezug auf ihre Qualität, d.h. die Erzeugung einer elektrisch leitenden Verbindung zwischen dem ersten Kontaktelement und dem zweiten Kontaktelement, geprüft werden.

In einer zu der U-förmigen bzw. zylindrischen Ausbildung des ersten Kontaktelements und des zweiten Kontaktelements alternativen Ausführungsform ist das erste Kontaktelement als erste metallische Folie und das zweite Kontaktelement als zweite metallische Folie ausgebildet. Der Isolator wird durch ein Keramik-Substrat gebildet. Das erste Kontaktelement und das zweite Kontaktelement sind ferner mit einem der Breite des Luftspalts entsprechenden Abstand nebeneinander auf dem Isolator angeordnet. Dieses Ausführungsbeispiel lässt sich besonders einfach und kostengünstig beispielsweise mittels Dickfilmtechnologie herstellen, wobei der Luftspalt vorzugsweise dadurch erzeugt wird, dass eine auf dem Keramik-Substrat liegende, durchgehende metallische Folie z.B. mittels Laserschneiden zertrennt wird. Hierdurch wird gleichzeitig die von der ersten metallischen Folie beabstandete zweite metallische Folie ausgebildet. Der elektrisch leitende Kontakt zu einem darunter liegenden elektrischen Schaltkreis (Leiterplatte, PCB) mit dem ersten Kontaktelement bzw. dem zweiten Kontaktelement erfolgt bei dieser Ausführungsform mittels einer seitlich an der Batteriebrücke angeordneten Lötbeschichtung, welche das Keramik-Substrat und vorzugsweise zusätzlich das darauf liegende erste Kontaktelement oder zweite Kontaktelement U-förmig umspannt. Zwischen dem ersten Kontaktelement und dem zweiten Kontaktelement bzw. dem Isolator und der Lötbeschichtung ist vorzugsweise eine Nickel-Zwischenschicht angeordnet, welche zur Herstellung der Lötbarkeit und Benetzbarkeit mit Weichlot dient.

Die schweißbare metallische Folie des ersten Kontaktelements und des zweiten Kontaktelements kann beispielsweise mindestens ein Material der Gruppe enthaltend Nickel, Kupfer, Kupfer-Nickel-Legierungen, Tantal, Niob, Molybdän, Titan und Edelstahl, aufweisen. Die Herstellung erfolgt im Dickschichtverfahren analog zu einem industriell gefertigten Dickschichtwiderstand (TFR-Thick Film Resistor). Aufgrund der Herstellung mittels Dickfilmtechnologie kann eine Batteriebrücke nach diesem Ausführungsbeispiel sehr effizient und preiswert erzeugt werden. Die metallische Folie des ersten Kontaktelements und/oder des zweiten Kontaktelements weist beispielsweise eine Schichtdicke im Bereich von 200 µm bis 600 µm auf. Die Abmessung einer nach diesem Ausführungsbeilspiel hergestellten Batteriebrücke kann etwa 2 mm x 1,25 mm betragen. Diese erfindungsgemäße Batteriebrücke zeichnet sich daher zusätzlich zu den oben angegebenen Eigenschaften durch kleine Abmessungen und daher durch einen geringen Flächenbedarf aus.

In einem weiteren alternativen Ausführungsbeispiel weist das erste Kontaktelement eine erste Form und das zweite Kontaktelement eine zweite Form auf, welche zu der ersten Form komplementär ist, wobei der Luftspalt an bzw. zwischen den gegenüberliegenden Flächen der ersten Form und der zweiten Form ausgebildet und in einem Teilbereich durch den Isolator ausgefüllt ist, der beispielsweise als Klebstofffilm (z.B. in Form eines Acrylatklebstoffs) ausgebildet ist. Das erste Kontaktelement und das zweite Kontaktelement sind schweißbare metallische Komponenten, aufweisend z.B. ein Material der Gruppe enthaltend Nickel, eine Kupfernickel-Legierung und Edelstahl. Als komplementäre Form kann beispielsweise eine L-Form (im Querschnitt) eingesetzt werden. Andere Formen, beispielsweise komplementäre Wellenformen, sind ebenfalls denkbar.

Der als Klebstofffilm ausgebildete Isolator, der im Bereich eines Teils des Luftspalts zwischen den beiden komplementären Formen angeordnet ist, bewirkt eine Fixierung des Luftspalts zwischen dem ersten Kontaktelement und dem zweiten Kontaktelement. Da sich das erste Kontaktelement und das zweite Kontaktelement bis zur Unterseite der Batteriebrücke erstrecken, können diese jeweils direkt mit einer Kontaktfläche eines darunter liegenden elektrischen Stromkreises (Leiterplatte) verbunden werden. Eine derartige Batteriebrücke besitzt eine Abmessung von beispielsweise 2 mm x 1,25 mm und hat daher einen geringen Platzbedarf. Die komplementären Formen des ersten und des zweiten Kontaktelements dieses Ausführungsbeispiels können durch Kaltverformen (Stanzen, Prägen) oder durch Materialabtrag (Fräsen, Wasserstrahlschneiden, Ätzen) hergestellt werden.

Vorzugsweise kann eine derartige Batteriebrücke mit einem mittels Kunststoffspritzguss hergestellten Gehäuse versehen sein. Hierbei liegt jedoch die Oberseite der Batteriebrücke mit dem ersten Kontaktelement, dem zweiten Kontaktelement und dem dazwischen angeordneten Luftspalt frei, so dass der Luftspalt für das Verschweißen bei der Aktivierung des elektrischen Schaltkreises zugänglich ist. Auch die Unterseite der Kontaktelemente ist nicht mit dem Gehäuse bedeckt, da diese zur Kontaktierung mit dem elektrischen Stromkreis dient.

Die obige Aufgabe wird ferner durch ein Verfahren zum Aktivieren einer elektronischen Vorrichtung, vorzugsweise eines elektronischen Implantats, mit einem elektrischen Schaltkreis mittels einer oben beschriebenen Batteriebrücke gelöst, welches die nachfolgenden Schritte aufweist:
- Positionieren und Befestigen der Batteriebrücke im ersten (offenen) Zustand auf dem elektrischen Schaltkreis sowie Herstellen einer elektrisch leitenden Verbindung zwischen dem ersten Kontaktelement und einer ersten Leiterbahn sowie zwischen dem zweiten Kontaktelement und einer zweiten Leiterbahn des elektrischen Schaltkreises, wobei die Verbindung mit der ersten Leiterbahn und/oder der zweiten Leiterbahn vorzugsweise über eine entsprechende Kontaktfläche erfolgt,
- Verbinden des elektrischen Schaltkreises mit einer Spannungsquelle und/oder einem Kondensator und/oder einem Dumpwiderstand,
- Hochfahren des elektrischen Schaltkreises über mindestens zwei vordefinierte Testpunkte,
- Überführen der Batteriebrücke in den zweiten (geschlossenen) Zustand durch zumindest abschnittsweises Verschweißen des ersten Kontaktelements und des zweiten Kontaktelements miteinander derart, dass der Luftspalt zumindest abschnittsweise dauerhaft elektrisch leitend verschlossen wird.

Das erfindungsgemäße Verfahren beinhaltet einen automatisierbaren Schweiß-Prozess und damit eine höhere Prozesssicherheit. Hierdurch kann die Durchlaufzeit für das Power-Up reduziert werden. Weiter sind bei dem erfindungsgemäßen Verfahren einfachere Fertigungshilfsmittel, beispielsweise lediglich ein Werkstückträger, verwendbar und keine zusätzlichen Teile zu- oder abzuführen, so dass der Fertigungsablauf geradliniger realisiert werden kann. Die erfindungsgemäße Batteriebrücke bzw. das erfindungsgemäße Verfahren kann daher insbesondere bei der Herstellung von elektronischen Implantaten im Bereich der Medizintechnik, insbesondere bei der Herzschrittmacher-/ICD/Biomonitor-Produktion eingesetzt werden.

Vorzugsweise wird zum Verschweißen ein Stumpfnahtschweißverfahren, ein Kehlnahtschweißverfahren oder ein Durchschweißverfahren eingesetzt, je nachdem welche Form der Luftspalt bzw. das erste Kontaktelement und das zweite Kontaktelement aufweisen.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den Ansprüchen oder deren Rückbeziehung.

Es zeigen schematisch:
- Fig. 1: ein erstes Ausführungsbeispiel einer erfindungsgemäßen Batteriebrücke im ersten Zustand in einer perspektivischen Ansicht von der Seite verbunden mit einer Leiterplatte,
- Fig. 2: die Anordnung gemäß Fig. 1 im Querschnitt,
- Fig. 3: das Ausführungsbeispiel gemäß Fig. 1 in einer Explosionsdarstellung in einer Ansicht von der Seite,
- Fig. 4: die Anordnung gemäß Fig. 3 im Querschnitt,
- Fig. 5 bis 9: ein erstes Ausführungsbeispiel eines erfindungsgemäßen Verfahrens zum Aktivieren einer elektronischen Vorrichtung mit einer Batteriebrücke gemäß Fig. 1 in mehreren Schritten, wobei in Fig. 5 bis 7 und 9 eine perspektivische Ansicht von der Seite und in Fig. 8 eine Schnittdarstellung gezeigt wird,
- Fig. 10: die Anordnung gemäß Fig. 1 im Querschnitt,
- Fig. 11: die Anordnung gemäß Fig. 10 in einem zweiten Zustand der Batteriebrücke,
- Fig. 12: ein zweites Ausführungsbeispiel einer erfindungsgemäßen Batteriebrücke in einem ersten Zustand in einer perspektivischen Ansicht von der Seite,
- Fig. 13: das Ausführungsbeispiel gemäß Fig. 12 im Querschnitt während des Verschweißens,
- Fig. 14: ein drittes Ausführungsbeispiel einer erfindungsgemäßen Batteriebrücke in einem ersten Zustand in einer perspektivischen Ansicht von der Seite,
- Fig. 15: das Ausführungsbeispiel gemäß Fig. 14 im Querschnitt während des Verschweißens,
- Fig. 16: ein viertes Ausführungsbeispiel einer erfindungsgemäßen Batteriebrücke in einer perspektivischen Ansicht von der Seite während des Verschweiβens,
- Fig. 17: ein fünftes Ausführungsbeispiel einer erfindungsgemäßen Batteriebrücke in einem ersten Zustand in einer perspektivischen Ansicht von der Seite verbunden mit einer Leiterplatte,
- Fig. 18: die Anordnung gemäß Fig. 17 im Querschnitt,
- Fig. 19: die Anordnung gemäß Fig. 18 in einem zweiten Zustand der Batteriebrücke,
- Fig. 20 bis 23: ein zweites Ausführungsbeispiel eines erfindungsgemäßen Verfahrens zum Aktivieren einer elektronischen Vorrichtung mit einer Batteriebrücke gemäß Fig. 17 in mehreren Schritten, jeweils in einer perspektivischen Ansicht von der Seite,
- Fig. 24: ein sechstes Ausführungsbeispiel einer erfindungsgemäßen Batteriebrücke in einem ersten Zustand in einer perspektivischen Ansicht von der Seite verbunden mit einer Leiterplatte,
- Fig. 25: die Anordnung gemäß Fig. 24 in einem zweiten Zustand der Batteriebrücke,
- Fig. 26: ein siebtes Ausführungsbeispiel einer erfindungsgemäßen Batteriebrücke in einem ersten Zustand in einer perspektivischen Ansicht von der Seite,
- Fig. 27: die Anordnung gemäß Fig. 26 in einem Querschnitt,
- Fig. 28: die Anordnung gemäß Fig. 26 in einem zweiten Zustand, in teilweiser transparenter Darstellung der Batteriebrücke und
- Fig. 29: eine elektronische Vorrichtung in Form eines Herzschrittmachers im Querschnitt einschließlich einer Schaltskizze mit einer erfindungsgemäßen Batteriebrücke gemäß Fig. 1 im ersten Zustand.

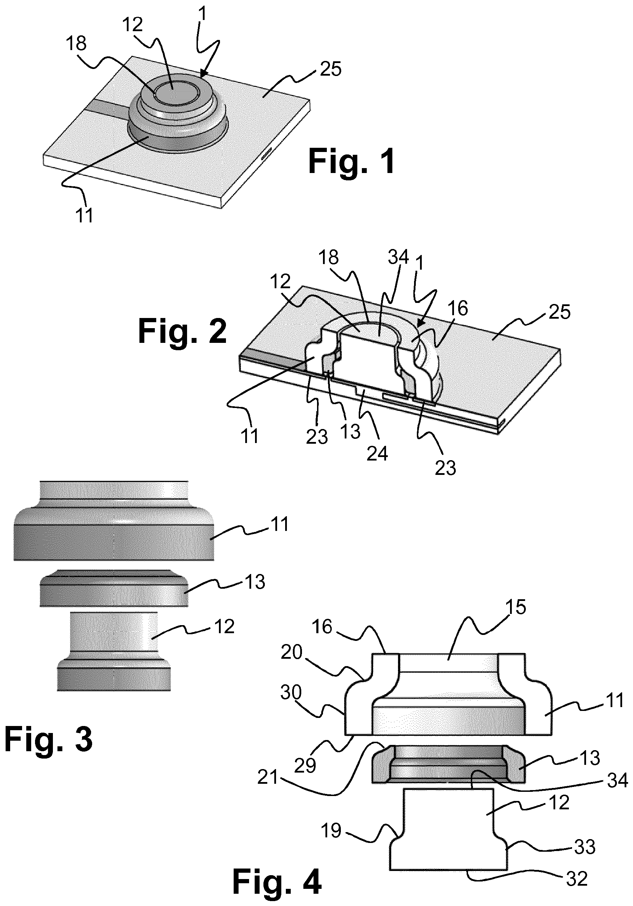

Das in den Fig. 1 bis 11 dargestellte erste Ausführungsbeispiel einer erfindungsgemäßen Batteriebrücke 1 weist eine im Wesentlichen schweißbare hohlzylinderförmige Kappe (erstes Kontaktelement) 11 und einen schweißbaren, im Wesentlichen zylinderförmigen Stempel (zweites Kontaktelement) 12 auf. Ferner ist ein Ring 13 (Isolator) vorgesehen, welcher zwischen der Kappe 11 und dem Stempel 12 angeordnet ist. Der Stempel 12 und der Ring 13 sind innerhalb der durchgehenden Öffnung 15 der Kappe 11 angeordnet. Der Ring 13 bewirkt eine konzentrische Positionierung von Stempel 12 und Kappe 11 derart zueinander, dass zwischen diesen ein Luftspalt 18 ausgebildet wird. Der Luftspalt hat beispielsweise eine Breite von etwa 50 µm. In dem in den Fig. 1, 2, 5, 6 und 10 dargestellten ersten (offenen) Zustand der Batteriebrücke sind die metallische Kappe 11 und der metallische Stempel 12 mittels des elektrisch isolierenden Rings 13 und des Luftspalts 18 voneinander elektrisch isoliert. Der Stempel 12 hat die gleiche Höhe wie die Kappe 11, so dass die obere Stirnfläche 16 der Kappe 11 und die obere Stirnfläche 34 des Stempels 12 miteinander fluchten.

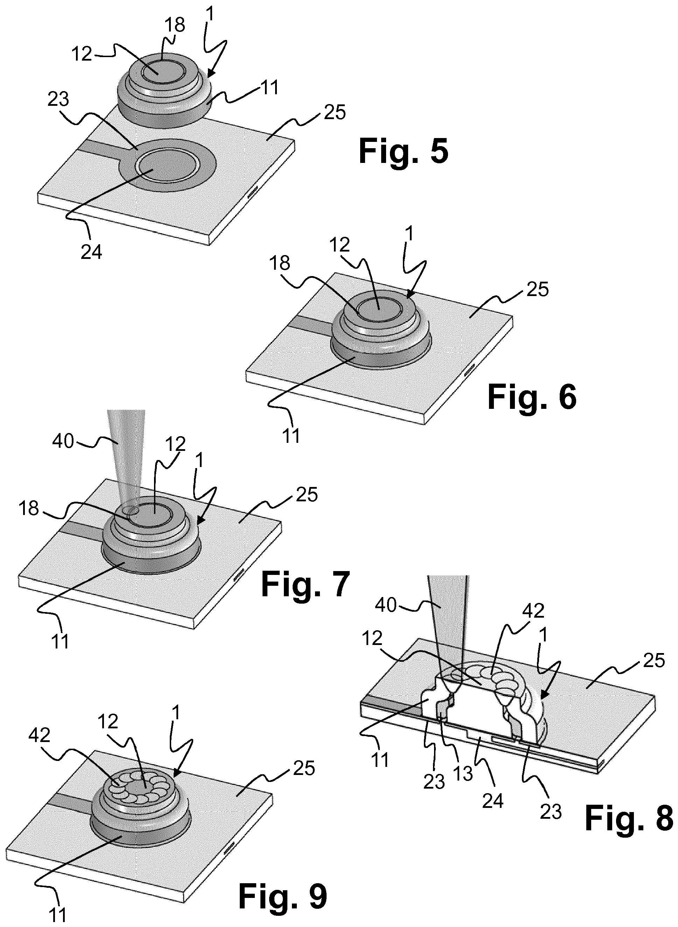

Der Stempel 12 weist in einem mittigen Bereich bezogen auf die Höhe der Batteriebrücke 1 bzw. des Stempels 12 einen abgeschrägten Abschnitt 19 auf, in dem sich der Durchmesser des Stempels 12 von einem kleineren Durchmesser auf einen größeren Durchmesser verändert. Die Durchmesseränderung liegt beispielsweise im Bereich zwischen 100 µm und 500 µm. In dem abgeschrägten Abschnitt 19 wird daher gewissermaßen ein Übergangsbereich oder "Knick" in dem Stempel 12 ausgebildet, welcher, wie in Fig. 8 dargestellt, Laserlicht eines Laserstrahls 40, der zum Verschweißen eingesetzt wird, von einem darunter liegenden elektrischen Schaltkreis - hier in der Form einer Leiterplatte 25 - abschirmt. Entsprechend kann auch die Kappe 11 einen abgeschrägten Abschnitt 20 und der Ring 13 einen abgeschrägten Abschnitt 21 aufweisen, wobei dieser Abschnitt 21 am Ring 13, wie Fig. 4 zeigt, im Bereich der Oberseite des Rings 13 angeordnet ist. Dies ergibt sich aufgrund der Anordnung des Rings 13 innerhalb der Kappe 11.

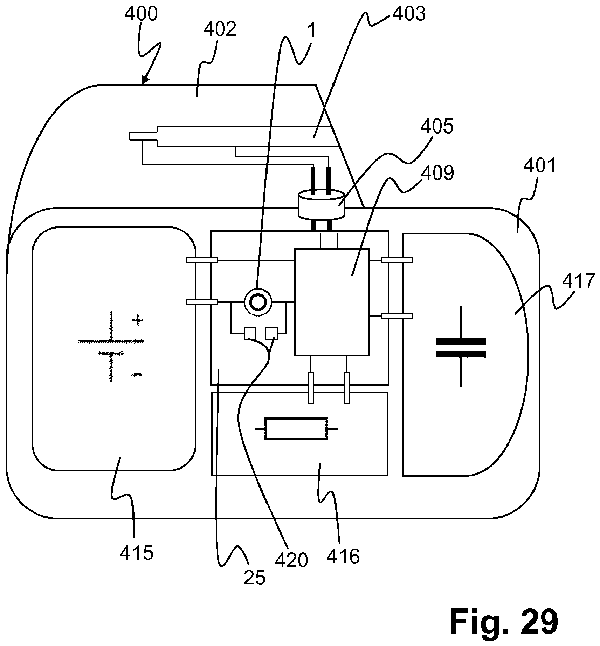

Nachfolgend soll nun anhand der Fig. 5 bis 9 und 29 das erfindungsgemäße Verfahren zum Aktivieren einer elektronischen Vorrichtung, beispielsweise eines elektronischen Implantats, mittels der Batteriebrücke 1 gemäß Fig. 1 und eines Herzschrittmachers 400 gemäß Fig. 29 als Beispiel für ein elektronisches Implantat erläutert werden.

Der Herzschrittmacher 400 weist ein nach außen hermetisch abgeschlossenes Gehäuse 401 und einen Header 402 mit einer Buchse 403 zur Herstellung der elektrisch leitenden Verbindung mit nicht dargestellten Elektroden auf. Die elektrische Verbindung von Kontakten in der Buchse mit der im Gehäuses 401 angeordneten Leiterplatte (PCB, printed circuit board) 25 und einem IC (integrierter Schaltkreis) 409 erfolgt mittels einer zwischen Header 402 und Gehäuse 401 angeordneten Durchführung 405, die ebenfalls nach außen hermetisch verschlossen ist. Wie Fig. 29 zu entnehmen ist, besitzt der IC 409 elektrisch leitende Verbindungen zu einer Batterie 415, einem Dumpwiderstand 416 und einer Kondensatoreinheit 417 mit einer Reihe von Kondensatoren, wobei die Batterie 415, der Dumpwiderstand 416 und die Kondensatoreinheit 417 im Gehäuse 401 des Herzschrittmachers 400 angeordnet sind. In der elektrischen Verbindung zwischen IC 409 und der Batterie 415 sind auf der Leiterplatte 25 die Batteriebrücke 1 und Testpunkte 420 parallel geschaltet angeordnet derart, dass die elektrische Verbindung zwischen Batterie 415 und IC 409 offen ist, solange sich die Batteriebrücke 1 im ersten (offenen) Zustand befindet.

Die Batteriebrücke 1 wird hergestellt, indem beispielsweise mittels eines Kaltverformungs- oder Stanzprozesses aus einem metallischen Material die Kappe 11 und der Stempel 12 jeweils separat gefertigt werden. Weiter wird beispielsweise mittels Kunststoffspritzgießen der Ring 13 produziert, beispielsweise bestehend aus POM, PEEK, LCP und/oder PBT. Nun wird zuerst der Ring 13 und anschließend der Stempel 12 in der durchgehenden Öffnung 15 der Kappe 11 angeordnet und eingepresst, sodass zwischen Kappe 11 und Ring 13 bzw. zwischen Ring 13 und Stempel 12 jeweils ein Presssitz ausgebildet wird. Hierdurch positioniert der Ring 13 die Kappe 11 und den Stempel 12 konzentrisch zueinander und verspannt diese miteinander, so dass der elektrisch isolierende Luftspalt 18 gleichmäßig ausgebildet wird. Die Batteriebrücke 1 befindet sich in dem ersten (offenen) Zustand.

Eine so hergestellte Batteriebrücke 1 wird nun, wie in Fig. 5 und 6 dargestellt, auf einer ersten Kontaktfläche 23 bzw. einer zweiten Kontaktfläche 24 der Leiterplatte 25 angeordnet und mit diesen elektrisch leitend, beispielsweise mittels Löten, verbunden. Hierfür kann an der Kappe 11 und/oder dem Stempel 12, jeweils an der unteren Stirnfläche 29 der Kappe 11, im unteren Bereich der Seitenfläche 30 der Kappe 11 bzw. an der unteren Stirnfläche 32 des Stempels 12 und/oder dem unteren Bereich der Seitenfläche 33 des Stempels 12, eine lötfähige Beschichtung (beispielsweise enthaltend mindestens ein Material der Gruppe aufweisend Gold, ENIG, EPIG, Palladium und Zinn) angeordnet sein, welche vor dem Zusammensetzen der Batteriebrücke 1 aufgebracht wurde. Durch das Löten wird hierbei die erste Kontaktfläche 23 mit der Kappe 11 elektrisch leitend und die zweite Kontaktfläche 24 mit dem Stempel 12 elektrisch leitend verbunden.

Nach Fertigstellung des Herzschrittmachers 400 mit dem zumindest teilweise als Leiterplatte 25 ausgeführten elektronischen Schaltkreis mit dem IC 409 und der Batteriebrücke 1 und nachdem alle Anschlüsse der Spannungsquelle (Batterie 415) und der Kondensatoreinheit 417 und des Dumpwiderstands 415 an dem Schaltkreis angeschlossen, vorzugsweise angeschweißt, worden sind, wird der Schaltkreis definiert über die zwei Testpunkte 420 initialisiert bzw. hochgefahren (Power-Up).

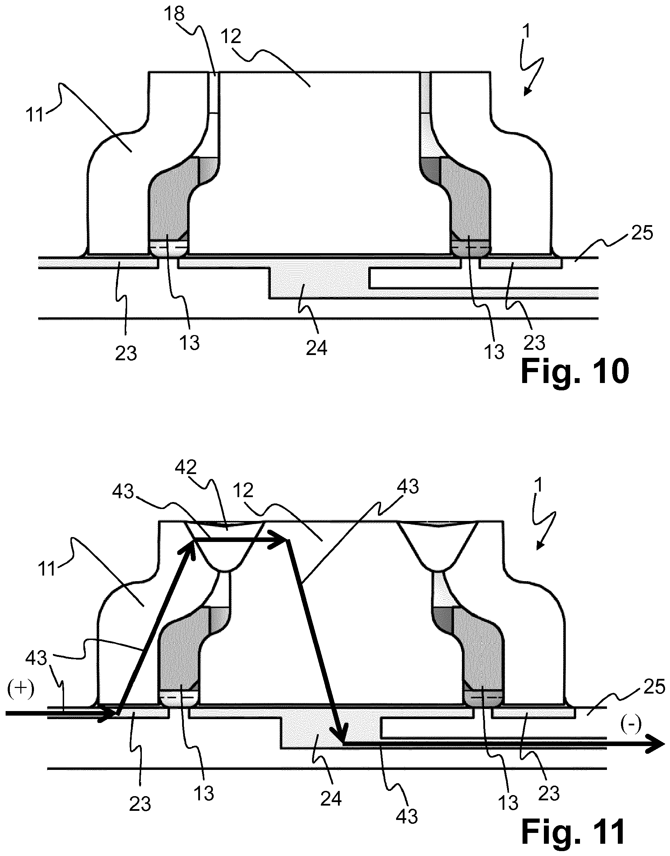

Nach erfolgreichem Power-Up können nun mit einem einzigen automatisierbaren Arbeitsschritt mittels Schweißen, beispielsweise mittels Laserschweißen, ohne weitere Materialzu- oder -abfuhr die Kappe 11 und der Stempel 12 der Batteriebrücke 1 miteinander verschmolzen werden. Dieser Arbeitsschritt ist in den Fig. 7 bis 9 gezeigt. Der Laserstrahl 40 wird hierfür im Bereich der Stirnfläche 16 der Kappe 11 entlang des Luftspalts 18 geführt und setzt Schweißpunkte, so dass im Bereich des Luftspalts 18 das Material der Kappe 11 und das Material des Stempels 12 miteinander verschmelzen, eine elektrisch leitende Verbindung zwischen Kappe 11 und Stempel 12 ausbilden und hierdurch den Luftspalt 18 durch eine Schweißnaht 42 auffüllen. Der Weg der Elektronen von einer ersten Leiterbahn mit der ersten Kontaktfläche 23 über die Kappe 11, die Schweißnaht 42, den Stempel 12 und die zweiten Kontaktfläche 24 einer zweiten Leiterbahn ist mittels Pfeilen 43 in Fig. 11 veranschaulicht. Der Stromkreis der Leiterplatte 25 wird somit permanent geschlossen und die Batteriebrücke 1 befindet sich im zweiten (geschlossenen) Zustand.

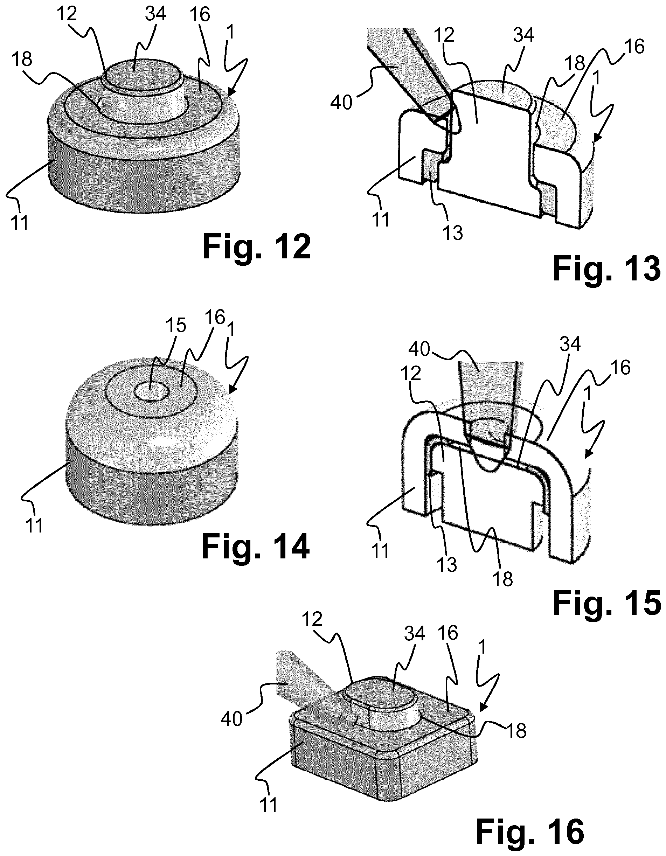

Bei dem in den Fig. 1 bis 11 dargestellten ersten Ausführungsbeispiel einer erfindungsgemäßen Batteriebrücke 1 entspricht die Höhe der Kappe 11 der Höhe des Stempels 12, so dass die obere Stirnfläche 34 des Stempels 12 bündig mit der Stirnfläche 16 der Kappe 11 verläuft. Demgegenüber ist bei dem in den Fig. 12 und 13 dargestellten zweiten Ausführungsbeispiel einer Batteriebrücke 1 die Höhe des Stempels 12 größer als die Höhe der Kappe 11, so dass der Stempel 12 über die Stirnfläche 16 der Kappe hinausragt. Bei diesem Ausführungsbeispiel ist der Laserstrahl 40 etwas einfacher zu positionieren, da er seitlich des Stempels 12 entlang des Luftspalts 18 geführt werden kann (siehe Fig. 13). Durch das Schweißen wird eine Kehlnaht im Bereich des Luftspalts 18 ausgebildet, welche die Kappe 11 mit dem Stempel 12 elektrisch leitend verbindet.

In Bezug auf das in den Fig. 1 bis 11 dargestellte erste Ausführungsbeispiel einer erfindungsgemäßen Batteriebrücke 1 besitzt nun bei dem in den Fig. 14 und 15 dargestellten dritten Ausführungsbeispiel der innen liegende Stempel 12 eine kleinere Höhe als die außen liegende Kappe 11. Die Kappe weist in ihrer Stirnfläche 16 eine durchgehende Öffnung 15 auf, durch die der Laserstrahl 40 hindurch geführt wird, um eine Verbindung zwischen dem Material der Kappe 11 und dem des Stempels 12 zu erreichen. Die Öffnung 15 der Kappe 11 kann auch durch eine Einkerbung ersetzt sein, also zumindest teilweise geschlossen ausgeführt sein, wobei dann durch die Einkerbung hindurch geschweißt wird, um die elektrisch leitende Verbindung mit dem Stempel 12 zu realisieren.

Fig. 16 zeigt ein viertes Ausführungsbeispiel einer erfindungsgemäßen Batteriebrücke, welches dem zweiten Ausführungsbeispiel gemäß Fig. 12 und 13 ähnelt. Die beiden Ausführungsbeispiele unterscheiden sind lediglich in der Form von Kappe 11, Stempel 12 bzw. (nicht dargestellt) Ring 13. Während diese Elemente bei dem zweiten Ausführungsbeispiel gemäß Fig. 12 und 13 rotationssymmetrisch ausgebildet sind, besitzt die Kappe 11 des Ausführungsbeispiels gemäß Fig. 16 eine nicht-rotationssymmetrische Form, nämlich eine Quaderform. Entsprechend ist der Stempel ebenfalls nicht kreiszylinderförmig ausgebildet. Die Querschnittsform ist hier im Wesentlichen eine Ellipse. Entsprechend ist auch der (nicht dargestellte) Isolator nicht als Kreisring, sondern eher als elliptischer/rechteckförmiger Ring realisiert. Dieses Ausführungsbeispiel hat den Vorteil, dass die Schweißnaht zumindest abschnittsweise linear verläuft. Weitere Formen sind für Kappe 11, Stempel 12 und Ring 13 der Batteriebrücke ebenfalls denkbar.

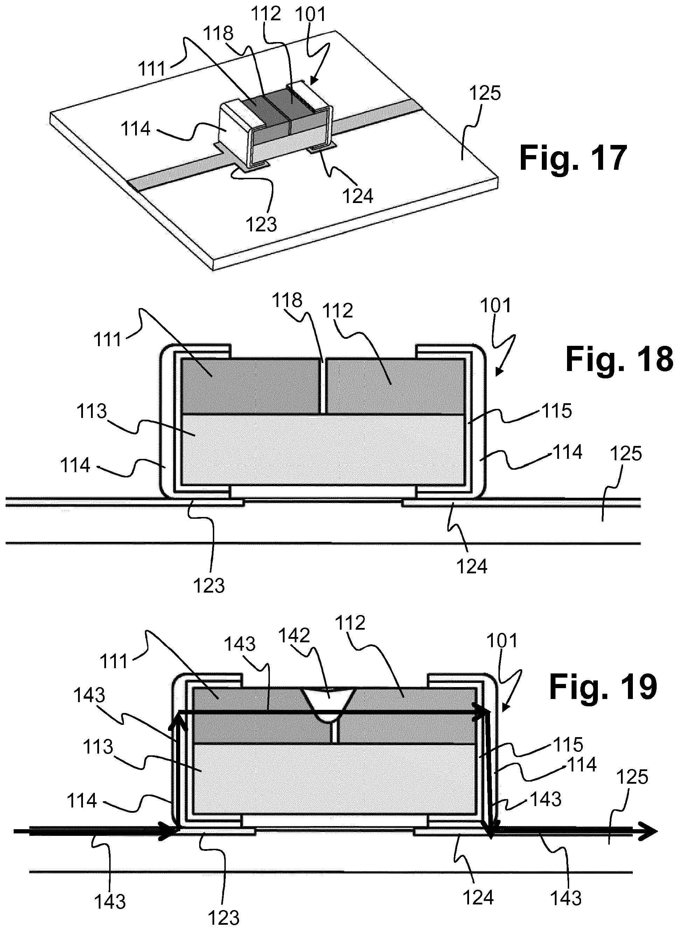

Alternativ zu der Herstellung der Kontaktelemente mittels Kaltverformungs- oder Stanzprozess wird nachfolgend anhand der Fig. 17 bis 23 ein fünftes Ausführungsbeispiel einer erfindungsgemäßen Batteriebrücke erläutert, bei dem die Komponenten mittels Dickfilmtechnologie hergestellt werden können, welche eine etablierte, sehr effiziente und preiswerte Technologie darstellt.

Die Batteriebrücke 101 weist eine erste schweißbare metallische Folie 111 (erstes Kontaktelement) und eine zweite schweißbare metallische Folie 112 (zweites Kontaktelement) auf, welche nebeneinander, getrennt durch einen Luftspalt 118 auf einem Keramik-Substrat (Isolator) 113 angeordnet sind. Die Dicke der metallischen Folien 111, 112 kann beispielsweise 400 µm und die Breite des Luftspalts kann z.B. 50 µm betragen. Die metallischen Folien 111, 112 können beispielsweise Nickel, Kupfer, eine Kupfer-NickelLegierung und/oder Edelstahl aufweisen oder aus einem oder mehreren dieser Materialien bestehen. Als Material für das Keramik-Substrat 113 kann beispielsweise LTCC (low temperature cofired ceramic) bestehend aus mehreren Werkstoffen (z.B. SiO₂, Glas, Titanate) oder HTCC (high temperature cofired ceramic mit der Hauptkomponente Al₂O₃) eingesetzt werden. Seitlich an der Batteriebrücke 101 ist jeweils eine Vorverzinnung/Lötbeschichtung 114 (im Folgenden kurz Lötbeschichtung 114) angeordnet, welche auf der einen Seite die erste metallische Folie 111 und das Keramik-Substrat 113 und auf der anderen Seite die zweite metallische Folie 112 und das Keramik-Substrat 113 U-förmig umschließt. Zwischen der Lötbeschichtung 114 und der jeweiligen metallischen Folie 111, 112 bzw. dem Keramik-Substrat 113 ist eine Nickel-Zwischenschicht 115 angeordnet. Durch die Lötbeschichtung 114 und die Nickel-Zwischenschicht 115 wird eine elektrisch leitende Verbindung von der jeweiligen metallischen Folie 111, 112 zur Unterseite des Keramik-Substrats 113 hergestellt.

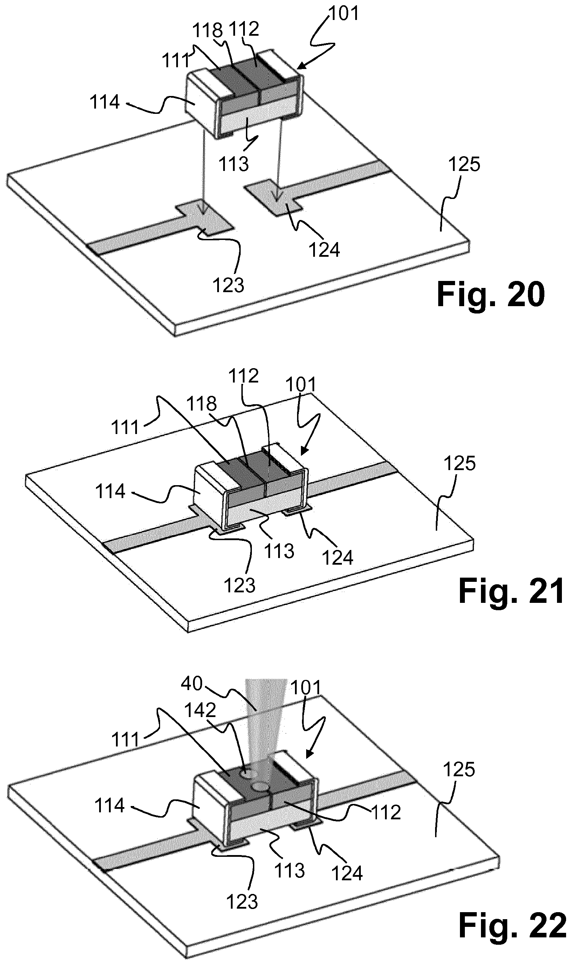

Das erfindungsgemäße Verfahren zur Aktivierung einer elektronischen Vorrichtung mit einer erfindungsgemäßen Batteriebrücke 101 gemäß dem fünften Ausführungsbeispiel erfolgt analog zu dem anhand der Fig. 5 bis 9 erläuterten Verfahren und ist in den Fig. 20 bis 23 dargestellt. Ein Unterschied besteht lediglich darin, dass bei der Batteriebrücke 101 nicht die Kontaktelemente 111, 112 direkt mit den Kontaktflächen 123, 124 der Leiterplatte 125 verbunden werden, sondern die jeweilige Lötbeschichtung 114, die den elektrischen Kontakt zu der oberhalb des Keramik-Substrats 113 angeordneten ersten metallischen Folie 111 bzw. zweiten metallischen Folie 112 herstellt.

Wie in Fig. 22 dargestellt wird, wird bei diesem Verfahren außerdem nicht die erste metallische Folie 111 mit der zweiten metallischen Folie 112 entlang des gesamten Luftspalts 118 verschmolzen, sondern es werden lediglich zwei entlang des Luftspalts 118 nebeneinanderliegenden Schweißpunkte 142 gesetzt. Alternativ kann natürlich lediglich ein Schweißpunkt oder es können mehr als zwei Schweißpunkte vorgesehen sein.

Der Weg der Elektronen entlang der Leiterbahn mit der ersten Kontaktfläche 123, der Lötbeschichtung 114, der ersten metallischen Folie 111, des Schweißpunkts 142, der zweiten metallischen Beschichtung 112, der Lötbeschichtung 114 und der Leiterbahn mit der zweiten Kontaktfläche 124 ist in Fig. 19 mittels Pfeilen 143 eingezeichnet.

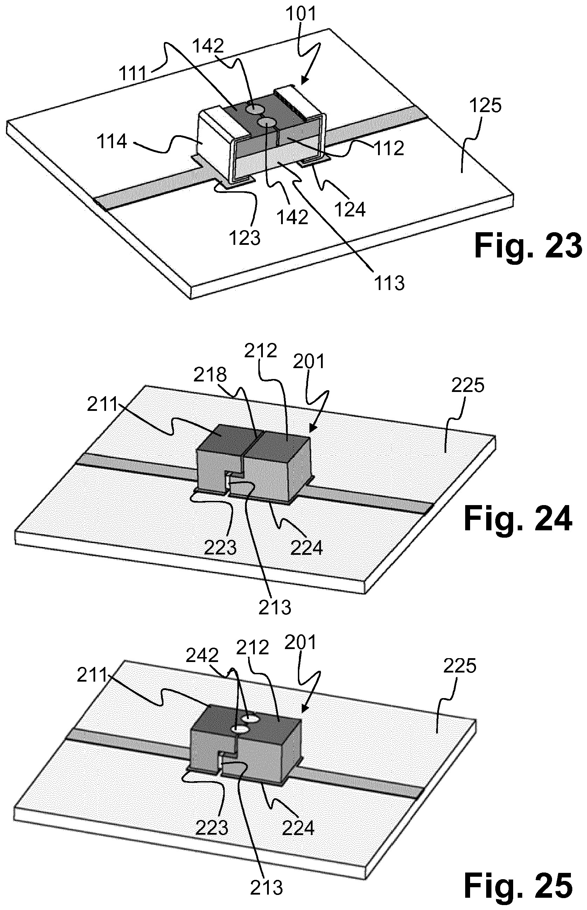

In den Fig. 24 und 25 ist ein sechstes Ausführungsbeispiel einer erfindungsgemäßen Batteriebrücke 201 gezeigt. Diese besitzt ein erstes Kontaktelement 211, das im Querschnitt eine L-Form aufweist, und ein zweites Kontaktelement 212, ebenfalls mit einer L-Form im Querschnitt. Diese komplementären Formen sind derart angeordnet, dass sie einander ergänzen und einen Luftspalt 218 zwischen dem ersten Kontaktelement 211 und dem zweiten Kontaktelement 212 ausbilden und zwar zwischen den einander gegenüberliegenden Flächen dieser Kontaktelemente 211, 212. Entsprechend bildet der Luftspalt 218 eine Stufenform aus. In einem unteren Bereich des Luftspalts 218 ist ein Isolator 213 in Form eines Klebstofffilms angeordnet, welcher beispielsweise streifenförmig ausgebildet ist. Die Kontaktelemente 211, 212 werden vorzugsweise mittels Kaltverformung, MIM (metal injection molding), Fräsen oder Ätzen und der Isolator 213 aus einem Acrylatklebstofffilm hergestellt. Die Anordnung auf einer Leiterplatte 225 mit einer ersten Kontaktfläche 223 und einer zweiten Kontaktfläche 224 erfolgt derart, dass das erste Kontaktelement 211 direkt mit der ersten Kontaktfläche 223 und das zweite Kontaktelement 212 direkt mit der zweiten Kontaktfläche 224 verlötet werden. Analog zu dem Ausführungsbeispiel der Batteriebrücke 111 wird eine Verbindung der beiden Kontaktelemente 211, 212 und der zweite (geschlossene) Zustand der Batteriebrücke 201 durch zwei Schweißpunkte 242 erreicht.

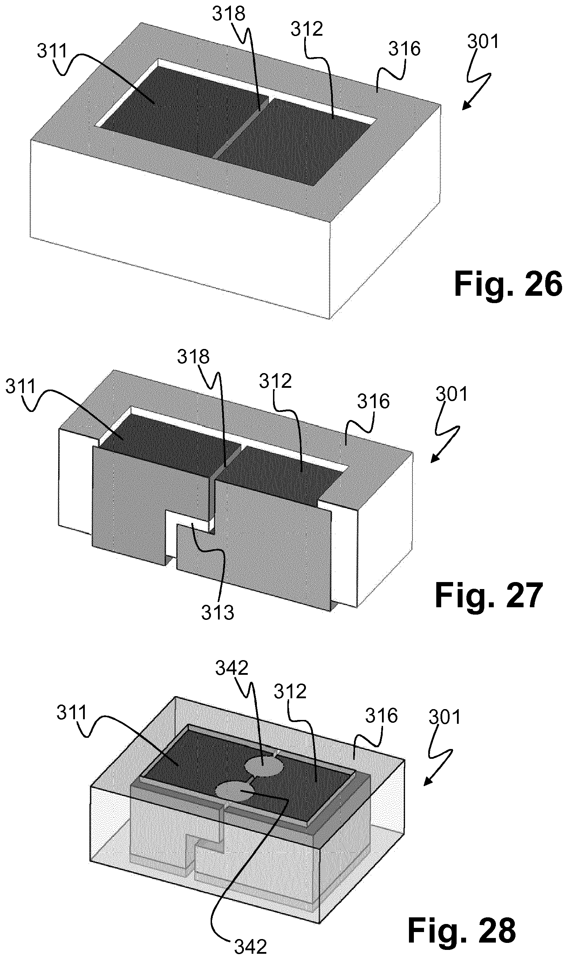

Das in den Fig. 26 bis 28 dargestellte siebente Ausführungsbeispiel einer erfindungsgemäßen Batteriebrücke 301 ähnelt im Aufbau der in den Fig. 24 und 25 dargestellten Batteriebrücke 201. Das erste Kontaktelement 311 und das zweite Kontaktelement 312 weisen ebenso jeweils eine im Querschnitt L-förmige komplementäre Form auf, zwischen denen ein Luftspalt 318 ausgebildet ist. Der beispielsweise als Klebstofffilm ausgebildete Isolator 313 besitzt hier ebenfalls im Querschnitt eine Streifenform, vorzugsweise mit einer Stufe. Weiter weist die Batteriebrücke 301 ein rechteckringförmiges Gehäuse 316 auf, welches das erste Kontaktelement 311 und das zweite Kontaktelement 312 ringförmig umgibt. An der Unterseite ragen das erste Kontaktelement 311 und das zweite Kontaktelement 312 über das Gehäuse 316 hinaus, um einen ordnungsgemäßen Kontakt zu entsprechenden Kontaktflächen einer Leiterplatte herstellen zu können. Bei dem in Fig. 28 dargestellten geschlossenen Zustand der Batteriebrücke 311 sind das erste Kontaktelement 311 und das zweite Kontaktelement 312 über Schweißpunkte 342 elektrisch leitend miteinander verbunden.

Die Vorteile der oben dargestellten erfindungsgemäßen SMT-fähige Batteriebrücken 1, 101, 201, 301 bzw. das oben erläuterte erfindungsgemäße Verfahren zum Aktivieren einer elektronischen Vorrichtung liegen insbesondere in der Automatisierbarkeit des Initialisierungs- und Schließprozesses des Stromkreises. Hierdurch kann eine höhere Prozesssicherheit und eine Reduzierung der Durchlaufzeit für das Power-Up erreicht werden. Es ist nicht notwendig, Material abzuführen und es ist ebenfalls nicht notwendig, Kontakte niederzuhalten. Mit einer erfindungsgemäß automatisiert schweißbaren Batteriebrücke 1, 101, 201, 301 kann der Fertigungsfluss stark vereinfacht werden.

### Bezugszeichenliste

- 1: Batteriebrücke
- 11: Kappe (erstes Kontaktelement)
- 12: Stempel (zweites Kontaktelement)
- 13: Ring (Isolator)
- 15: Öffnung
- 16: obere Stirnfläche der Kappe 11
- 18: Luftspalt
- 19: Abschnitt
- 20: Abschnitt
- 21: Abschnitt
- 23: erste Kontaktfläche einer Leiterbahn
- 24: zweite Kontaktfläche einer Leiterbahn
- 25: Leiterplatte
- 29: untere Stirnfläche der Kappe 11
- 30: Seitenfläche der Kappe 11
- 32: untere Stirnfläche des Stempels 12
- 33: Seitenfläche des Stempels 12
- 34: obere Stirnfläche des Stempels 12
- 40: Laserstrahl
- 42: Schweißnaht
- 43: Pfeil

- 101: Batteriebrücke
- 111: metallische Folie (erstes Kontaktelement)
- 112: metallische Folie (zweites Kontaktelement)
- 113: Keramik-Substrat (Isolator)
- 114: Vorverzinnung/Lötbeschichtung
- 115: Zwischenschicht
- 118: Luftspalt
- 123: erste Kontaktfläche einer Leiterbahn
- 124: zweite Kontaktfläche einer Leiterbahn
- 125: Leiterplatte
- 142: Schweißpunkt
- 143: Pfeil

- 201: Batteriebrücke
- 211: erstes Kontaktelement
- 212: zweites Kontaktelement
- 213: Isolator
- 218: Luftspalt
- 223: erste Kontaktfläche einer Leiterbahn
- 224: zweite Kontaktfläche einer Leiterbahn
- 225: Leiterplatte
- 242: Schweißpunkt

- 301: Batteriebrücke
- 311: erstes Kontaktelement
- 312: zweites Kontaktelement
- 313: Isolator
- 316: Gehäuse
- 318: Luftspalt
- 342: Schweißpunkt

- 400: Herzschrittmacher
- 401: Gehäuse
- 402: Header
- 403: Buchse
- 405: Durchführung
- 409: IC
- 415: Batterie
- 416: Dumpwiderstand
- 417: Kondensatoreinheit
- 420: Testpunkt

## Patentansprüche

1. Batteriebrücke (1, 101, 201, 301) für eine elektronische Vorrichtung, vorzugsweise für ein elektronisches Implantat, umfassend ein elektrisch leitendes erstes Kontaktelement (11, 111, 211, 311), ein elektrisch leitendes zweites Kontaktelement (12, 112, 212, 312) und einen
Isolator (13, 113, 213, 313),
wobei in einem ersten Zustand der Batteriebrücke das erste Kontaktelement über einen vorgegebenen Luftspalt (18, 118, 218, 318) beabstandet von dem zweiten Kontaktelement angeordnet ist, **dadurch gekennzeichnet, dass**
das erste Kontaktelement durch den Luftspalt und den Isolator elektrisch von dem zweiten Kontaktelement isoliert ist,
wobei das erste Kontaktelement und das zweite Kontaktelement ein schweißbares Material aufweisen, wobei die Batteriebrücke derart ausgebildet ist, dass sie mittels Verschweißen des ersten Kontaktelements und des zweiten Kontaktelements miteinander in einen zweiten Zustand überführbar ist, bei dem der Luftspalt zwischen dem ersten Kontaktelement und dem zweiten Kontaktelement zumindest abschnittsweise elektrisch leitend geschlossen ist.

2. Batteriebrücke (1) nach Anspruch 1, **dadurch gekennzeichnet, dass** der Isolator (13) im Wesentlichen ringförmig, das erste Kontaktelement (11) im Querschnitt im Wesentlichen U-förmig oder hohlzylinderförmig und das zweiten Kontaktelement (12) im Wesentlichen zylinderförmig ausgebildet ist, wobei der ringförmige Isolator zwischen dem ersten Kontaktelement und dem zweiten Kontaktelement angeordnet ist.

3. Batteriebrücke (1) nach Anspruch 2, **dadurch gekennzeichnet, dass** das zweite Kontaktelement (12) innerhalb einer innenliegenden Öffnung (15) des ersten Kontaktelements (11), welche vorzugsweise durchgehend ist, angeordnet ist.

4. Batteriebrücke (1) nach einem der Ansprüche 2 und 3, **dadurch gekennzeichnet, dass** das zweite Kontaktelement (12) einen abgeschrägten Abschnitt (19) aufweist, welcher ein zum Verschweißen verwendetes Laserlicht von einem unterhalb der Batteriebrücke angeordneten elektrischen Schaltkreis (25) abschirmt.

5. Batteriebrücke (1) nach einem der Ansprüche 2 bis 4, **dadurch gekennzeichnet, dass** das erste Kontaktelement (11) und/oder das zweite Kontaktelement (12) in einem zur Verbindung mit dem elektrischen Schaltkreis (25) dienenden Abschnitt eine die Lötbarkeit verbessernde Beschichtung aufweist.

6. Batteriebrücke (1) nach einem der Ansprüche 2 bis 5, **dadurch gekennzeichnet, dass** das zweite Kontaktelement (12) im Bereich einer oberen Stirnfläche (16) des ersten Kontaktelements (11), auf der das Verschweißen erfolgt, über diese obere Stirnfläche hinausragt.

7. Batteriebrücke (101) nach Anspruch 1, **dadurch gekennzeichnet, dass** der Isolator als Keramik-Substrat (113) sowie das erste Kontaktelement als erste metallische Folie (111) und das zweite Kontaktelement als zweite metallische Folie (112) ausgebildet ist, wobei das erste Kontaktelement und das zweite Kontaktelement mit einem der Breite des Luftspalts (118) entsprechenden Abstand auf dem Isolator angeordnet sind.

8. Batteriebrücke (201, 301) nach Anspruch 1, **dadurch gekennzeichnet, dass** das erste Kontaktelement (211, 311) eine erste Form und das zweite Kontaktelement (212, 312) eine zweite Form aufweist, welche zu der ersten Form komplementär ist, wobei der Luftspalt (218, 318) an den gegenüber liegenden Flächen der ersten Form und der zweiten Form ausgebildet und in einem Teilbereich durch den Isolator ausgefüllt ist, der beispielsweise als Klebstofffilm ausgebildet ist.

9. Batteriebrücke nach einem der vorhergehenden Ansprüche, **dadurch gekennzeichnet, dass** der Luftspalt (18, 118, 218, 318) eine Breite im Bereich von 10 µm bis 100 µm, vorzugsweise im Bereich von 30 µm bis 80 µm, aufweist.

10. Verfahren zum Aktivieren einer elektronischen Vorrichtung (400), vorzugsweise eines elektronischen Implantats, mit einem elektrischen Schaltkreis mittels einer Batteriebrücke (1, 101, 201, 301) nach einem der vorhergehenden Ansprüche **gekennzeichnet durch** die nachfolgenden Schritte:
- Positionieren und Befestigen der Batteriebrücke im ersten Zustand auf dem elektrischen Schaltkreis (25, 125, 225) sowie Herstellen einer elektrisch leitenden Verbindung zwischen dem ersten Kontaktelement (11, 111, 211, 311) und einer ersten Leiterbahn sowie zwischen dem zweiten Kontaktelement (12, 112, 212, 312) und einer zweiten Leiterbahn des elektrischen Schaltkreises,
- Verbinden des elektrischen Schaltkreises mit einer Spannungsquelle (415) und/oder einem Kondensator (417) und/oder einem Dumpwiderstand (416),
- Hochfahren des elektrischen Schaltkreises über mindestens zwei vordefinierte Testpunkte (420),
- Überführen der Batteriebrücke in den zweiten Zustand durch zumindest abschnittsweises Verschweißen des ersten Kontaktelements und des zweiten Kontaktelements miteinander derart, dass der Luftspalt (18, 118, 218, 318) zumindest abschnittsweise elektrisch leitend verschlossen wird.

11. Verfahren nach Anspruch 10, **dadurch gekennzeichnet, dass** zum Verschweißen ein Stumpfnahtschweißverfahren oder ein Kehlnahtschweißverfahren oder ein Durchschweißverfahren eingesetzt wird.

## Claims

1. A battery bridge (1, 101, 201, 301) for an electronic device, preferably, for an electronic implant, comprising an electrically conductive first contact element (11, 111, 211, 311), an electrically conductive second contact element (12, 112, 212, 312), and an insulator (13, 113, 213, 313), ,
wherein, in a first state of the battery bridge, the first contact element is distanced from the second contact element via a predefined air gap (18, 118, 218, 318) **characterized in that**
the first contact element is electrically insulated from the second contact element by the air gap and the insulator, wherein the first contact element and the second contact element comprise a weldable material, and
wherein the battery bridge is configured in such a way that it can be transferred, by welding the first contact element and the second contact element to one another, into a second state, in which the air gap between the first contact element and the second contact element is closed electrically conductively, at least in part.

2. The battery bridge (1) according to claim 1, **characterised in that** the insulator (13) is substantially annular, the first contact element (11) is substantially U-shaped or hollow cylindrical in cross-section, and the second contact element (12) is substantially cylindrical, wherein the annular insulator is arranged between the first contact element and the second contact element.

3. The battery bridge (1) according to claim 2, **characterised in that** the second contact element (12) is arranged within an inner opening (15) of the first contact element (11), which opening is preferably continuous.

4. The battery bridge (1) according to either one of claims 2 or 3, **characterised in that** the second contact element (12) has a sloped portion (19), which shields an electric circuit (25) arranged beneath the battery bridge from a laser light used for welding.

5. The battery bridge (1) according to any one of claims 2 to 4, **characterised in that** the first contact element (11) and/or the second contact element (12) has, in a portion serving for connection to the electric circuit (25), a coating which improves solderability.

6. The battery bridge (1) according to any one of claims 2 to 5, **characterised in that** the second contact element (12), in the region of an upper end face (16) of the first contact element (11), in which region the welding is performed, protrudes beyond this upper end face.

7. The battery bridge (101) according to claim 1, **characterised in that** the insulator is formed as a ceramic substrate (113) and the first contact element is formed as a first metal foil (111) and the second contact element is formed as a second metal foil (112), wherein the first contact element and the second contact element are arranged on the insulator at a distance corresponding to the width of the air gap (118).

8. The battery bridge (201, 301) according to claim 1, **characterised in that** the first contact element (211, 311) has a first shape and the second contact element (212, 312) has a second shape, which is complementary to the first shape, wherein the air gap (218, 318) is formed at the opposing faces of the first shape and the second shape and in a region is filled out by the insulator, which is formed for example as an adhesive film.

9. The battery bridge according to any one of the preceding claims, **characterised in that** the air gap (18, 118, 218, 318) has a width ranging from 10 µm to 100 µm, preferably ranging from 30 µm to 80 µm.

10. A method for activating an electronic device (400), preferably an electronic implant, comprising an electric circuit by means of a battery bridge (1, 101, 201, 301) according to any one of the preceding claims, **characterised by** the following steps:
- positioning and fastening the battery bridge in the first state on the electric circuit (25, 125, 225) and producing an electrically conductive connection between the first contact element (11, 111, 211, 311) and a first conductive track and between the second contact element (12, 112, 212, 312) and a second conductive track of the electric circuit,
- connecting the electric circuit to a voltage source (415) and/or a capacitor (417) and/or a dump resistor (416),
- powering up the electric circuit via at least two predefined test points (420),
- transferring the battery bridge into the second state by at least partially welding the first contact element and the second contact element to one another, in such a way that the air gap (18, 118, 218, 318) is electrically conductively closed, at least in part.

11. The method according to claim 10, **characterised in that** a butt seam welding method or a fillet welding method or a through-welding method is used for welding.

## Revendications

1. Elément de liaison pour batterie (1, 101, 201, 301) destiné à un dispositif électronique, de préférence à un implant électronique, comprenant un premier élément de contact (11, 111, 211, 311) électriquement conducteur, un deuxième élément de contact (12, 112, 212, 312) électriquement conducteur et un isolant (13, 113, 213, 313),
où, dans un premier état de l'élément de liaison pour batterie, le premier élément de contact est disposé espacé par rapport au deuxième élément de contact par le biais d'un intervalle d'air (18, 118, 218, 318) prédéfini,
**caractérisé en ce que**
le premier élément de contact est isolé électriquement par rapport au deuxième élément de contact par l'intervalle d'air et l'isolant,
où le premier élément de contact et le deuxième élément de contact présentent un matériau soudable, où l'élément de liaison pour batterie est conçu de telle façon qu'il puisse être transposé dans un deuxième état au moyen du soudage conjoint du premier élément de contact avec le deuxième élément de contact, état dans lequel l'intervalle d'air entre le premier élément de contact et le deuxième élément de contact est fermé au moins par endroits en étant électriquement conducteur.

2. Elément de liaison pour batterie (1) selon la revendication 1, **caractérisé en ce que** l'isolant (13) est conçu dans l'ensemble de forme annulaire, le premier élément de contact (11) est conçu dans l'ensemble en forme de U ou en forme de cylindre creux dans la section transversale et le deuxième élément de contact (12) est conçu dans l'ensemble en forme de cylindre, où l'isolant de forme annulaire est disposé entre le premier élément de contact et le deuxième élément de contact.

3. Elément de liaison pour batterie (1) selon la revendication 2, **caractérisé en ce que** le deuxième élément de contact (12) est disposé à l'intérieur d'un orifice (15) du premier élément de contact (11) se situant à l'intérieur, lequel va de préférence de part en part.

4. Elément de liaison pour batterie (1) selon l'une des revendications 2 et 3, **caractérisé en ce que** le deuxième élément de contact présente un segment (19) biseauté, lequel abrite une lumière laser employée pour le soudage d'un circuit (25) électrique disposé en-dessous de l'élément de liaison pour batterie.

5. Elément de liaison pour batterie (1) selon l'une des revendications 2 à 4, **caractérisé en ce que** le premier élément de contact (11) et/ou le deuxième élément de contact (2) présentent un revêtement améliorant la soudabilité dans un segment servant à la liaison avec le circuit (25) électrique.

6. Elément de liaison pour batterie (1) selon l'une des revendications 2 à 5, **caractérisé en ce que** le deuxième élément de contact (12), dans la région d'une surface frontale supérieure (16) du premier élément de contact (11), dépasse au-dessus de cette surface frontale supérieure sur laquelle a lieu le soudage.

7. Elément de liaison pour batterie (1) selon la revendication 1, **caractérisé en ce que** l'isolant est conçu sous forme de substrat en céramique (113), ainsi que le premier élément de contact est conçu sous forme d'un premier film (111) métallique et le deuxième élément de contact est conçu sous forme de deuxième film (112) métallique, où le premier élément de contact et le deuxième élément de contact sont disposés sur l'isolant sur une distance correspondant à la largeur de l'intervalle d'air (118).

8. Elément de liaison pour batterie (1) selon la revendication 1, **caractérisé en ce que** le premier élément de contact (211, 311) présente une première forme et le deuxième élément de contact (212, 312) présente une deuxième forme, laquelle est complémentaire à la première forme, où l'intervalle d'air (218, 318) est conçu sur les surfaces de la première forme et de la deuxième forme se situant en face et est rempli dans une zone partielle par l'isolant qui est par exemple conçu sous forme d'un film d'adhésif.

9. Elément de liaison pour batterie (1) selon l'une des revendications précédentes, **caractérisé en ce que** l'intervalle d'air (18, 118, 218, 318) présente une largeur dans la plage de 10 µm à 100 µm, de préférence, dans la plage de 30 µm à 80 µm.

10. Procédé d'activation d'un dispositif (400) électronique, de préférence, d'un implant électronique, avec un circuit électrique, au moyen d'un élément de liaison pour batterie (1, 101, 201, 301) selon l'une des revendications précédentes, **caractérisé par** les étapes suivantes :
- le positionnement et la fixation de l'élément de liaison pour batterie dans un premier état sur le circuit électrique (25, 125, 225), ainsi que l'établissement d'une liaison électriquement conductrice entre le premier élément de contact (11, 111, 211, 311) et une première piste conductrice ainsi qu'entre le deuxième élément de contact (12, 112, 212, 312) et une deuxième piste conductrice du circuit électrique,
- la connexion du circuit électrique avec une source de tension (415) et/ou un condensateur (417), et/ou une résistance de décharge (416),
- le démarrage du circuit électrique par le biais d'au moins deux points de test (420),
- la transposition de l'élément de liaison pour batterie dans le deuxième état par un soudage conjoint au moins par endroits du premier élément de contact et du deuxième élément de contact de telle manière que l'intervalle d'air (18, 118, 218, 318) est fermé en étant électriquement conducteur au moins par endroits.

11. Procédé selon la revendication 10, **caractérisé en ce qu'**un procédé de soudage en bout à bout ou un procédé de soudage d'angle, ou un procédé de soudage à cœur est mis en œuvre pour le soudage.
